# EUROPEAN PATENT APPLICATION

(11) **EP 4 414 936 A1**
(43) Date of publication of application: **14.08.2024**
(21) Application number: 23156143.2
(22) Date of filing: 10.02.2023
(51) Int. Cl.: G06T 7/33, A61B 5/00, A61B 8/13, A61B 8/08

(54) **REGISTERING 3D IMAGE DATA**

(71) Applicant: Koninklijke Philips N.V., 5656 AG Eindhoven (NL)
(72) Inventor: GROTH, Alexandra, Eindhoven (NL); WEBER, Frank Michael, Eindhoven (NL); VAN GEFFEN, Michel, 5656AG Eindhoven (NL)
(74) Representative: Philips Intellectual Property & Standards

(57) **Abstract**

The present invention relates to registering second 3D image data to first 3D image data. In order to provide an improved combination of different 3D image data, a device (10) for registering second 3D image data to first 3D image data is provided that comprises a data input (12), a data processor (14) and an output interface (16). The data input is configured to provide first 3D image data comprising a first mesh with a first topology; and to provide second 3D image data comprising a second mesh with a second topology, wherein the second topology is different than the first topology, and wherein one of the first mesh and the second mesh is a to-be-converted mesh and the other of the first mesh and the second mesh is a conversion-target mesh. The data input is also configured to provide a conversion model to convert the to-be-converted mesh to the conversion-target mesh and to provide a plurality of weights for the to-be-converted mesh. The data processor is configured to convert the to-be-converted mesh to the conversion-target mesh based on the conversion model; wherein, based on the conversion model, the plurality of weights for the to-be-converted mesh is converted into weights for the conversion-target mesh to thereby generate a plurality of converted weights. The output interface is configured to provide the plurality of converted weights for further image data processing.

## Description

### FIELD OF THE INVENTION

The present invention relates to a device for registering second 3D image data to first 3D image data, to a medical imaging system and to a method for registering second 3D image data to first 3D image data.

### BACKGROUND OF THE INVENTION

In medical imaging, as an example, ultrasound imaging is used by sonographers to diagnose e.g. various cardiac conditions. In contrast to other medical imaging modalities, ultrasound imaging has advantages like the absence of ionizing radiation. Further, ultrasound imaging can be operated in a non-invasive, non-intrusive manner. Therefore, ultrasound imaging is often used during interventional procedures or for clinical follow-ups. However, ultrasound images have a lower resolution than CT (computed tomography, e.g. X-ray CT) or MR (magnetic resonance) images. Therefore, ultrasound images are less anatomically detailed. For interventional planning, often CT or MR images are preferred over ultrasound images. On the other hand, during interventional procedures or for clinical follow-ups, CT or MR imaging is rather unpractical. In order to be able to provide enhanced information during interventional procedures or for clinical follow-ups, pre-interventional images can be combined with interventional images, which requires a number of image processing steps. In combining images, not only the image itself, but also information derived from these images like the outlines of anatomical structures are displayed to the physician or further processed, such as measurements, abstract visualizations or (bio-mechanical) modeling. An example is the overlay of extracted anatomical outlines, the images themselves or planning results of pre-interventional images, e.g. CT or MR, over interventional images, e.g., ultrasound images, to enrich the live images with additional information, for example for navigation. However, it has been shown that the combining may also result in combining errors of the respective imaging.

### SUMMARY OF THE INVENTION

There may thus be a need to provide an improved combination of different 3D image data.

The object of the present invention is solved by the subject-matter of the independent claims; further embodiments are incorporated in the dependent claims. It should be noted that the following described aspects of the invention apply also for the device for registering second 3D image data to first 3D image data, for the medical imaging system and for the method for registering second 3D image data to first 3D image data.

According to the present invention, a device for registering second 3D image data to first 3D image data is provided. The device comprises a data input, a data processor and an output interface. The data input is configured to provide first 3D image data comprising a first mesh with a first topology. The data input is also configured to provide second 3D image data comprising a second mesh with a second topology. The second topology is different than the first topology. One of the first mesh and the second mesh is a to-be-converted mesh and the other of the first mesh and the second mesh is a conversion-target mesh. The data input is further configured to provide a conversion model to convert the to-be-converted mesh to the conversion-target mesh. The data input is furthermore configured to provide a plurality of weights for the to-be-converted mesh. The data processor is configured to convert the to-be-converted mesh to the conversion-target mesh based on the conversion model. Based on the conversion model, the plurality of weights for the to-be-converted mesh is converted into weights for the conversion-target mesh to thereby generate a plurality of converted weights. The output interface is configured to provide the plurality of converted weights for further image data processing.

As an advantageous result, the weights for both meshes are provided in the same topology. They can be combined in the same way as weights combined in each of the topologies.

The workflow can be used for systems requiring mesh or pre-acquired data overlays on interventional live images.

According to an example, the first 3D image data is different than the second 3D image data. A difference is provided for at least one of the group comprising: image data resolution, time of image acquisition, subject's state for image acquisition, imaging used for generating the image data and imaging modality used for generating the image data.

According to an example, the data input is configured to provide the first 3D image data as interventional 3D image data, and to provide the second 3D image data as pre-interventional 3D image data.

According to an example, the data processor is configured to assess image quality of both the second 3D image data and the first 3D image data separately. The data processor is configured to compute an overall weight distribution based on both weight distributions of the second 3D image data and the first 3D image data.

According to an example, the data processor is configured to derive the plurality of weights from imaging affected parameters of the actual imaging of the second 3D image data and/or the first 3D image data, the parameters comprising at least one of the group of: image quality and field of view.

According to an example, the data processor is configured to derive the plurality of weights from systematic inter-modality deviations of the imaging modalities.

According to an example, the data processor is configured to derive the plurality of weights from patient specific circumstances.

According to an example, the data processor is configured to calculate different weight distributions with different methods. The output interface is configured to display the different weight distributions to the user for selection. The data processor is configured to apply the selected method for conversion.

According to an example, the data processor is configured to register the first mesh with the second mesh based on the conversion model and the plurality of the converted weights. The data processor is also configured to register the second 3D image data and the first 3D image data based on the registering of the first mesh and the second mesh, to thereby generate combined image data. The output interface is configured to present the combined image data.

According to the present invention, also a medical imaging system is provided. The system comprises a first imaging modality and a device for registering second 3D image data to first 3D image data according to one of the preceding examples. The first imaging modality provides the first 3D image data.

According to an example, it is provided a second imaging modality. The second imaging modality provides second 3D image data. Further, the second imaging modality is different than the first imaging modality.

According to the present invention, also a method for registering second 3D image data to first 3D image data is provided. The method comprises the following steps:
- Providing first 3D image data comprising a first mesh with a first topology; and providing second 3D image data comprising a second mesh with a second topology; wherein the second topology is different than the first topology; and wherein one of the first mesh and the second mesh is a to-be-converted mesh and the other of the first mesh and the second mesh is a conversion-target mesh;
- Providing a conversion model to convert the to-be-converted mesh to the conversion-target mesh;
- Providing a plurality of weights for the to-be-converted mesh;
- Converting the to-be-converted mesh to the conversion-target mesh based on the conversion model; wherein, based on the conversion model, the plurality of weights for the to-be-converted mesh is converted into weights for the conversion-target mesh thereby generating a plurality of converted weights; and
- Providing the plurality of converted weights for further image data processing.

According to an aspect, converting weights for a mesh from one topology into another topology based on the conversion model provides the advantage that the registration of both image data is improved. As an example, if the topology of the meshes to be registered are different from each other, and the vertex-to-vertex correspondences are not known upfront, the mesh correspondence is provided by the conversion step. This avoids e.g. the need to estimate a correspondence on-the-fly during registration.

Using the particular conversion model also results in that the registration is optimized by taken into account the respective situation. The conversion model addresses that calculated correspondences are not universally valid and can change from case to case and even from acquisition to acquisition for the same patient. As the registration result might be influenced by the current mesh shape, the conversion model provides the benefit to avoid inconsistent results between different patients or even different acquisitions from the same patient.

Converting weights for a mesh from one topology into another topology based on the conversion model provides a registration method that establishes the correspondences between meshes independently for the individual case.

According to an aspect, meshes with different topologies are registered to each other and one of the meshes is converted into the topology of the second mesh via the so-called conversion model. As this results in knowledge of the vertex-to-vertex correspondence, as further steps, registration techniques are provided.

In model-based segmentation, so-called conversion models can convert a loaded mesh into another topology. A conversion model consists of at least two hierarchical levels with mean meshes of the topologies in question. As in every segmentation model, the relations of the different hierarchical levels are encoded in the model as a vertex correspondence lists. Therefore, by switching between different hierarchical levels, a loaded mesh can be transferred to and read out in any of the encoded mean mesh topologies of the model. Topology mapping via conversion model is possible in model-based segmentation because the segmented mesh has the same topology as the trained mean mesh of the segmentation model.

As an example, the correspondence between topologies is a direct vertex-to-vertex correspondence. That means that one mesh comprises a subset of vertices from the other mesh. Vertices of the finer mesh without a counterpart in the coarser mesh are interpolated by the surrounding vertices. If the correspondence between topologies is not a direct vertex-to-vertex correspondence (vertices do not have a direct counterpart in the other topology), the closest vertex in the second topology is assigned in the vertex correspondence list. This would lead to an error which is smaller the finer the second mesh is, since the distances between the real vertex and the replacement vertex is small. However, by converting weights for a mesh from one topology into another topology based on the conversion model, the error is avoided or at least minimized.

Converting weights also addresses that during mesh registration in practice there are vertex pairs that have smaller error than others. During registration that is considered by assigning different registration weights for each vertex pair. Thereby, the higher the weight for a vertex pair is, the smaller the registration error for this specific vertex pair in correspondence to the remaining vertex pairs. The weights defined for the vertices of the mesh to be registered, are converted into the topology of the target mesh.

To derive weights from imaging-related content reflects the different reasons for the definition of higher or lower vertex weights for registration, e.g. due to the image quality, the patient disease, or the image modalities. For example, mesh vertices that are not in the field-of-view (in the original image during its segmentation) may have weights so that they are not considered during registration. To obtain smaller errors for certain vertex pairs, weights are introduced for registration.

The idea of a conversion module aims at providing simple models which are easier in handling, e.g. when registering different images. A so-to-speak normal model may have edges and points in space but contains usually also more features. For conversion, a two-fold or multi-fold level of a segmentation module is provided, while making use of the level theory. In a conversion module, information used in the context of the segmentation of the image data is left aside, resulting in a reduced data set.

One option for a topology is triangular meshes, comprising vertices and lines, which are coded in a mesh. Such triangular mesh based topology is characterized by the number of its elements and their relative positions, i.e. their interconnections. For example, image data from CT imaging may be used for achieving a topology that is different than a topology achieved from image data from ultrasound imaging. It is noted that CT and ultrasound are one example for combining information from different imaging with different topologies.

As an example, a live image is used to extract a mesh and to convert the mesh to a mesh of a CT image, instead of directly registering the image itself to an image from CT.

When applying a model based segmentation, topology is not altered. The particular concept of weights and their transfer based on the conversion module provided in the present context leads to an improved combination of the two different images. In other words, weights used registration are mapped. The weights are based on additional information about for example distortion or the like. Such additional information provides an additional layer of information. The converted weights no longer relate to components of the (source) topology, but to the target topology. The weights are converted from one topology to another.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for registering second 3D image data to first 3D image data.
Fig. 2 shows an example of a medical imaging system with a device for registering second 3D image data to first 3D image data.
Fig. 3 shows basic steps of an example of a method for registering second 3D image data to first 3D image data.
Fig. 4 shows a working scheme of another example for registering second 3D image data to first 3D image data.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

As an example of 3D image data, in 3D, the anatomical outlines are represented by meshes, e.g. triangular meshes consisting of vertices and triangles, and are extracted from medical images by segmentation algorithms like model-based segmentation. To register the pre-interventional 3D mesh to an interventional image, the interventional image can be segmented, and the segmentation result can be registered to the pre-interventional mesh. This provides a mesh-to-mesh instead of a mesh-to-image registration. For the segmentation of the interventional (live) image, a segmentation model with a coarser topology can be used to enhance the segmentation speed (and the segmentation result is not visualized anyway).

In general, it cannot be assumed for mesh-to-mesh registration that meshes have the same topology. Reasons for different topologies can be manifold, such as different anatomical details or subparts are required, for example heart with and without valves, or different image modality resolutions, for example, since CT is a modality with a higher resolution than ultrasound, meshes can also have a finer topology.

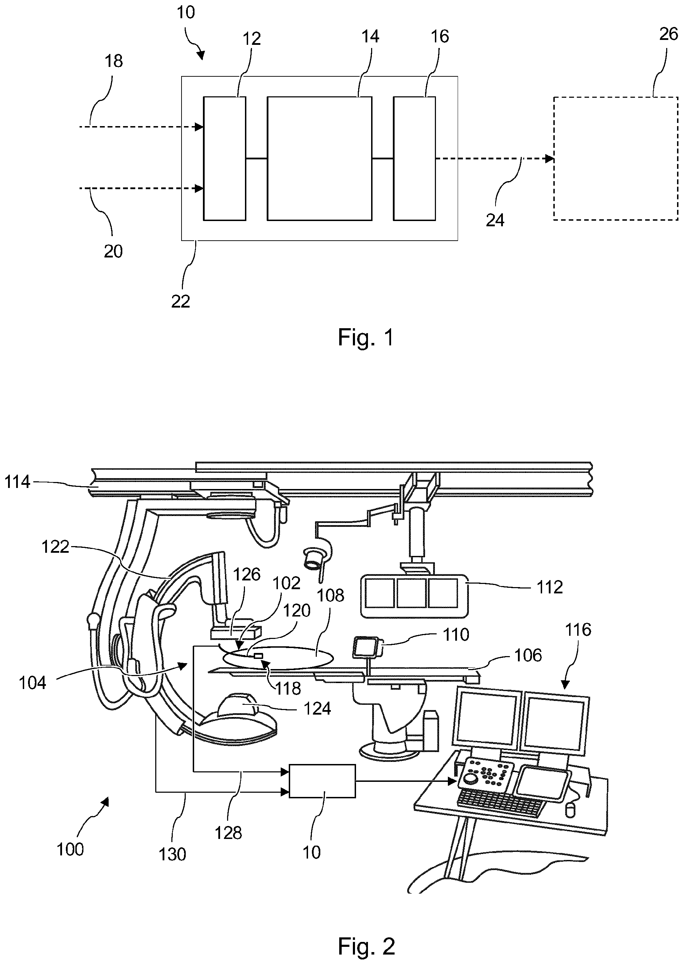

Fig. 1 schematically shows an example of a device 10 for registering second 3D image data to first 3D image data. The device 10 comprises a data input 12, a data processor 14 an output interface 16. The data input 12 is configured to provide first 3D image data comprising a first mesh with a first topology. The data input 12 is also configured to provide second 3D image data comprising a second mesh with a second topology. The second topology is different than the first topology; one of the first mesh and the second mesh is a to-be-converted mesh and the other of the first mesh and the second mesh is a conversion-target mesh. The data input 12 is also configured to provide a conversion model to convert the to-be-converted mesh to the conversion-target mesh and to provide a plurality of weights for the to-be-converted mesh. The data processor 14 is configured to convert the to-be-converted mesh to the conversion-target mesh based on the conversion model. Based on the conversion model, the plurality of weights for the to-be-converted mesh is converted into weights for the conversion-target mesh to thereby generate a plurality of converted weights. The output interface 16 is configured to provide the plurality of converted weights for further image data processing.

A first arrow 18 in broken lines indicates the provision of the first 3D image data. A second arrow 20 in broken lines indicates the provision of the second 3D image data. In an example, the data is provided from one or several data storages. In another example, the data is provided from one or several imaging arrangements. In a further example, the data is provided from a combination of at least one data storage and at least one imaging arrangement.

A frame 22 indicates an option according to which the data input 12, the data processor 14 and the output interface 16 are provided in an integrated manner, e.g. in a common housing. In another option, the data input 12, the data processor 14 and the output interface 16 are at least partly provided in a separated manner.

A third arrow 24 in broken lines indicates the provision of the plurality of converted weights for further processing, such as for being shown on a monitor or display device, indicated with a further frame 26 shown in broken lines.

In other words, the conversion model is provided to convert the to-be-converted mesh to the conversion-target mesh and also to convert the weights. Before being converted, the weights are to-be-converted weights. Once being converted, the weights are converted weights.

The term "data input" relates to providing or supplying data for data processing steps. The data input 12 can also be referred to as image data input. The data input 12 can also be referred to as data supply, as image data supply, as image input, as input unit or simply as input. As an example, the data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. As an example, the data processor 14 is data-connected to the data input 12 and the output interface 16.

The term "output interface" relates to an interface for providing the processed or computed data for further purposes. The output interface 16 can also be referred to as output or output unit. As an example, the output interface 16 is data-connectable to a display arrangement or display device. As another example, the output interface 16 is data-connected to a display. As an example, the signals by the controller can be provided by the output interface 16.

As an example, the different image data resolution relates to a resolution of the first image data being different than the resolution of the second image data. For example, the resolution of the second image data is higher than the resolution of the first image data.

As an example, the different time of image acquisition relates to a time point, time span or time frame when the second image data is acquired and a time point, time span or time frame when the first image data is acquired. The time difference may be coupled to states of an intervention or treatment plan, or may be coupled to a check-up or comparing schedule.

As an example, the different subject's state for image acquisition relates to the physiological state of the subject. For example, the subject is in a pre-interventional state during the acquisition of the second image data and in a post- or inter-interventional state during the acquisition of the first image data. As another example, the subject is in a non-treated state during the acquisition of the second image data and in a treated or at least partly treated state during the acquisition of the first image data. As another example, the subject is in a non-prepared state during the acquisition of the second image data and in a prepared or at least partly prepared state during the acquisition of the first image data. For example, the subject is contrast-injected during the acquisition of one of the first or second image data and non-contrast-injected in the other one.

The term "subject" may also be referred to as individual. The "subject" may further also be referred to as patient, although it is noted that this term does not indicate whether any illness or disease is actually present with the subject.

As an example, the imaging used for generating the image data relates to the physical imaging principle applied for generating the image data. For example, ultrasound imaging is provided for generating one of the first or second image data; and X-ray imaging is provided for generating the other one of the first or second image data.

As an example, the imaging modality used for generating the image data relates to different imaging devices. For example, a mobile X-ray imaging device, like a fluoro imager, is provided for generating one of the first or second image data; and a stationary X-ray device, like an X-ray C-arm or CT system, is provided for generating the other one of the first or second image data.

In an option of the example of Fig. 1, not further shown in detail though, the first 3D image data is different than the second 3D image data. A difference is provided for at least one of the group comprising: image data resolution, time of image acquisition, subject's state for image acquisition, imaging used for generating the image data and imaging modality used for generating the image data.

In an option of the example of Fig. 1, not further shown in detail, the data input 12 is configured to provide the first 3D image data as interventional 3D image data. The data input 12 is also configured to provide the second 3D image data as pre-interventional 3D image data.

As an example, the plurality of weights is derived from imaging-related content. For example, the plurality of weights provides an additional layer of information.

As an example, the plurality of weights provides an additional layer of information.

In an option of the example of Fig. 1, not further shown in detail, the data processor 14 is configured to assess image quality of both the second 3D image data and the first 3D image data separately. The data processor 14 is also configured to compute an overall weight distribution based on both weight distributions of the second 3D image data and the first 3D image data.

In an option of the example of Fig. 1, not further shown in detail, the data processor 14 is configured to derive the plurality of weights from imaging affected parameters of the actual imaging of the second 3D image data and/or the first 3D image data, the parameters comprising at least one of the group of image quality and field of view.

In an option of the example of Fig. 1, not further shown in detail, the data processor 14 is configured to derive the plurality of weights from systematic inter-modality deviations of the imaging modalities.

In an example, the plurality of weights reflects information about modality related distortions.

In an option of the example of Fig. 1, not further shown in detail, the data processor 14 is configured to derive the plurality of weights from patient specific circumstances.

In an option of the example of Fig. 1, not further shown in detail, the data processor 14 is configured to derive the plurality of weights from different types of the image-related content. The data processor 14 is also configured to generate a separate set of a plurality of weights for each type for each image category of the second 3D image data and the first 3D image data. The data processor 14 is further configured to transfer the different sets of the plurality of weights of each image category into a common plurality of weights for each image category.

In an option of the example of Fig. 1, not further shown in detail, the data processor 14 is configured to generate a graphical indicator for the plurality of converted weights. The output interface 16 is configured to show the graphical indicator overlaid to a representation of the target conversion-mesh.

In an option of the example of Fig. 1, not further shown in detail, the data processor 14 is configured to calculate different weight distributions with different methods. A display, like the monitor 26, is provided that is configured to display the different weight distributions to the user for selection. The data processor 14 is configured to apply the selected method for conversion.

In an option of the example of Fig. 1, not further shown in detail, the data processor 14 is configured to register the first mesh with the second mesh based on the conversion model and the plurality of the converted weights. The data processor 14 is also configured to register the second 3D image data and the first 3D image data based on the registering of the first mesh and the second mesh, to thereby generate combined image data. The output interface 16 is configured to present the combined image data.

Fig. 2 shows an example of a medical imaging system 100 comprising a first imaging modality 102 and an example of the device 10 for registering second 3D image data to first 3D image data according to one of the preceding examples. The first imaging modality 102 provides the first 3D image data.

As an example, the first imaging modality 102 provides interventional image data.
In an option of Fig. 2, it is provided a second imaging modality 104. The second imaging modality 104 provides second 3D image data. The second imaging modality 104 is different than the first imaging modality 102.

Fig. 2 shows the medical imaging system 100 in the context of a cathlab setting. For example, a subject support 106 is provided and a subject 108 is arranged thereon. A bedside control interface 110 is indicated as well as some monitor arrangement 112 above, suspended from a ceiling mounted rail structure 114. Further, a console 116 is shown in the foreground.

The first imaging modality 102 is shown as an intraluminal ultrasound imaging device 118 mounted at the distal end of a catheter 120. The second imaging modality 104 is shown as a C-arm structure with a C-arm 122 movably mounted to the ceiling mounted rail structure 114. An X-ray source 124 is mounted to one end of the C-arm 122, and an X-ray detector 126 is mounted to the other end of the C-arm 122.

A first arrow 128 indicates the provision of the first 3D image data to the device 10 for registering second 3D image data to first 3D image data. A second arrow 130 indicates the provision of the second 3D image data to the device 10 for registering second 3D image data to first 3D image data. As an example, the second imaging modality 104 provides pre-interventional 3D image data.

The first 3D image data, e.g. the interventional image data, can be referred to as first images. The second 3D image data, e.g. the pre-interventional 3D image data, can be referred to as second images.

As an example, the first 3D image data, e.g. the interventional image data, is data from a first imaging procedure and the second 3D image data, e.g. the pre-interventional 3D image data, is data from a second imaging procedure. The first and the second imaging procedures are different in their setup and concept.

As an example, the first imaging procedure is ultrasound imaging, and the second imaging procedure is CT imaging.

As another example, the first imaging procedure is fluoroscopy imaging, and the second imaging procedure is CT imaging.

As a further example, the first imaging procedure is ultrasound imaging, and the second imaging procedure is MR imaging.

As a still further example, the first imaging procedure is fluoroscopy imaging, and the second imaging procedure is MR imaging.

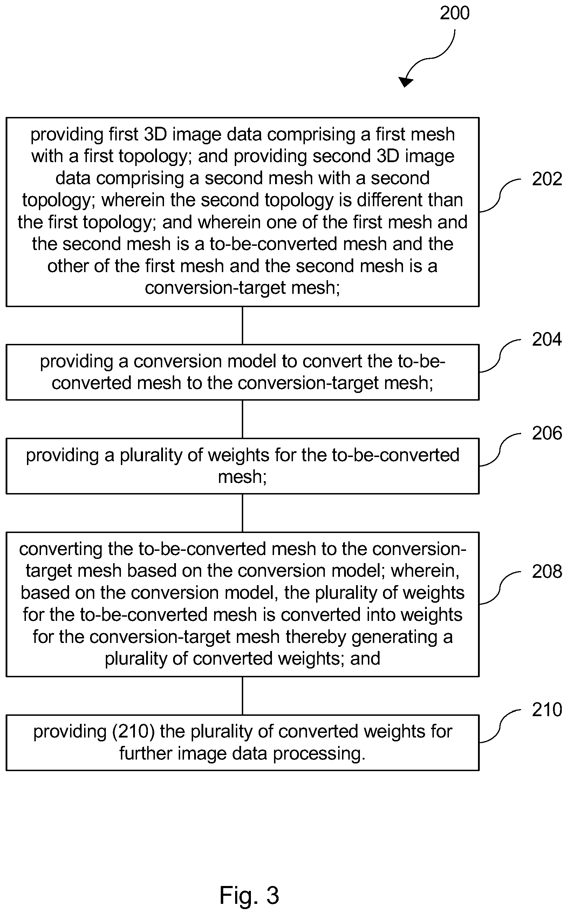

Fig. 3 shows basic steps of an example of a method 200 for registering second 3D image data to first 3D image data. The method 200 comprises the following steps:
In a first step 202, first 3D image data is provided in a first sub-step comprising a first mesh with a first topology. Further, second 3D image data is provided in a second sub-step comprising a second mesh with a second topology. The second topology is different than the first topology. One of the first mesh and the second mesh is a to-be-converted mesh and the other of the first mesh and the second mesh is a conversion-target mesh.
In a second step 204, a conversion model to convert the to-be-converted mesh to the conversion-target mesh is provided.
In a third step 206, a plurality of weights for the to-be-converted mesh is provided.
In a fourth step 208, the to-be-converted mesh is converted to the conversion-target mesh based on the conversion model; based on the conversion model, the plurality of weights for the to-be-converted mesh is converted into weights for the conversion-target mesh thereby generating a plurality of converted weights.
In a fifth step 210, the plurality of converted weights is provided for further image data processing.

As an example, the first 3D image data, e.g. the interventional image data is provided, and the first mesh is generated based on the first 3D image data.

As an example, the second 3D image data, e.g. the pre-interventional 3D image data is provided, and the second mesh is generated based on the second 3D image data.

The conversion model is a model-based segmentation conversion model.

The conversion-target mesh can also be referred to as target mesh.

As an example, the pre-interventional (e.g. second) 3D data is 3D CT image data. The interventional (e.g. first) 3D image data is ultrasound image data.

As an example, the vertex weights used for registration with a conversion model are converted from the mesh topology of the mesh to be registered into the mesh topology of the target mesh. This provides a weight transfer.

Converting the weights provides a weight transfer from the one mesh to the other mesh, i.e. from the to-be-converted mesh to the conversion-target mesh.

As a first example, the conversion model is provided to convert the first mesh of the first (e.g. interventional) 3D image data to the second mesh of the second (e.g. pre-interventional) 3D image data. The first mesh is then converted to the second mesh based on the conversion model. The conversion model can also be referred to as first-mesh conversion model, first conversion model or first-to-second conversion model. The weights of the first topology are converted into weights of the second topology.

As a second example, the conversion model is provided to convert the second mesh of the second (e.g. pre-interventional) 3D image data to the first mesh of the first (e.g. interventional) 3D image data. The second mesh is then converted to the first mesh based on the conversion model. The conversion model can also be referred to as second-mesh conversion model, second conversion model or second-to-first conversion model. The weights of the second topology are converted into weights of the first topology

As an example, the plurality of converted weights is combined with the registration weight defined for vertices of the conversion-target mesh.

As an example, the known weights for a mesh topology to be registered are transferred into the target topology via the conversion model. As an option, for vertices with a vertex-to-vertex correspondence for the different hierarchy levels, the weights can be directly transferred. For vertices that do not have a match in the previous hierarchy level, the weight is interpolated according to the mesh connections, e.g. from the nearest vertex with weight, or via linear interpolation from the N nearest neighbors.

As an example, a conversion model is a degenerated segmentation model, since other elements of a segmentation model, like the boundary detectors, might not be implemented in the model as they are not required for the conversion task.

In an example of the method, the first 3D image data is different than the second 3D image data; and a difference is provided for at least one of the group comprising: image data resolution, time of image acquisition, subject's state for image acquisition, imaging used for generating the image data, and imaging modality used for generating the image data.

In an example of the method, the first 3D image data is provided as interventional 3D image data; the second 3D image data is provided as pre-interventional 3D image data.

In an example of the method, the plurality of weights is derived from imaging-related content; and the plurality of weights provides an additional layer of information.

In an example of the method, image quality of both the second 3D image data and the first 3D image data are assessed separately; an overall weight distribution is computed based on both weight distributions of the second 3D image data and the first 3D image data.

As an example, image quality of both the pre-interventional 3D image data and the interventional 3D image data are assessed separately; an overall weight distribution is computed based on both weight distributions of the second 3D image data and the first 3D image data.

As an example, vertex weights are defined in a different manner. A combination of both weight distributions to an overall distribution is required.

In an example of the method according to claim 14, 15 or 16, wherein the plurality of weights is derived from imaging affected parameters of the actual imaging of the second 3D image data and/or the first 3D image data, the parameters comprising at least one of the group of: image quality and field of view.

As an example, the plurality of weights is derived from imaging affected parameters of the actual imaging of the pre-interventional 3D image data and/or the interventional 3D image data, the parameters comprising at least one of the group of: image quality and field of view.

The weights can be referred to as image-acquisition-specific weights.

As an example, based on the image quality, certain images areas can be defined where the segmentation results are expected to be error-prone or of less accuracy, e.g. due to artefacts, for example metal artifacts in CT. As an option, the weights, e.g. the vertex weights, are lower in these areas than in areas of normal image quality. As another option, a region-based automatic image quality assessment assesses, i.e. give votes for image quality, and a vertex weight is multiplied with these votes. The votes can also be referred to as factors.

As another example for an imaging affecting parameter in the criteria of image quality is that the field-view is smaller than the imaged organ. Due to the limited field-of-view, certain mesh parts are out of image and are interpolated according to the prior-knowledge encoded in the mean mesh. The vertex weights of these out-of-scope vertices should be set to zero for registration. Adjacent vertices still in the image might be affected during segmentation by the reduced field-of-view.

As an option, a transition region from the field-of-view inwards is defined. Vertices in this transition region have linearly reduced vertex weights, e.g. from "1" (one) to "0" (zero).

In an example of the method, the plurality of weights is derived from systematic inter-modality deviations of the imaging modalities.

The weights can be referred to as modality-specific weights.

In an example of the method, the plurality of weights reflects information about modality related distortions.

As an example, the second, e.g. pre-interventional, 3D image data and the first, e.g. interventional, 3D image data are provided by different image modalities and each image modality has a distinct, e.g. local, distortion pattern. As an option, these specific local image distortion patterns are learned, and weights are trained. Areas that have a high image distortion are assigned lower weighted in the registration procedure. For example, the atria are longer in TEE (Transesophageal echocardiography) than the biological ground truth; the actual heart and atria vertices should therefore have a lower weight. By later combining the modified vertex weights of both images, the weights for the inter-modality distortions are learned. Another reason for distortions might be the different patient position for different modalities. As an example, for a CT scan, the patient is lying with the arms up to the head, whereas in TEE ultrasound, the arms are down. This also leads to a systematic deviation in the organ forms. Adapting the weights by conversion addresses this and provides improved image data, i.e. increased accuracy.

As an option, inter-modality distortions weights are encoded in the conversion model.

As an example, training the inter-modality weights directly without a combination of weights for different modalities is provided wherein the weights reflect if the distortions of both modalities are different for a certain vertex.

As an advantage, this aims at a relevant aspect of the registration procedure. As an example for registration, it is not relevant if vertices show a large distortion to the ground truth if both distortions behave in the same way.

In an example of the method, the plurality of weights is derived from patient specific circumstances.

The weights can be referred to as patient-specific weights.

As an example, several images of the same patient at different time instances are provided, and certain anatomical areas that change continuously over time and that are, therefore, less reliable for registration, are anticipated from previous images. Such areas can be assigned with a weighting factor for adapted mesh registration.

As an example, the registration of meshes from different follow-ups for the examination of the progression of a certain disease, e.g. heart failure with thicker myocardia at the beginning of the disease and larger ventricles during its progression, is provided. Such anatomical related aspects can be assigned with a weighting factor for adapted mesh registration.

As another example, no prior image, but a prior diagnosis is available, e.g. malfunction of the aortic valve, is provided. The less reliable areas are identified by the anatomical model labels and weights can be lowered in these areas.

As a further example, weights are defined by prior knowledge about the interventions and their effects on inter-interventional images in contrast to pre-interventional images.

In an example of the method, the plurality of weights is derived from different types of the image-related content, a separate set of a plurality of weights is generated for each type for each image category of the second 3D image data and the first 3D image data; and the different sets of the plurality of weights of each image category are transferred into a common plurality of weights for each image category.

As an example, a multiplication for the different sets of the plurality of weights of each image category is generated to provide the common plurality of weights; e.g. a multiplication of all known weights for a vertex.

As another example, a weighted average of the different sets of the plurality of weights of each image category is generated to provide the common plurality of weights.

As a further example, the lowest weight of all known weights for a vertex of the different sets of the plurality of weights of each image category is generated to provide the common plurality of weights.

In an example of the method, a graphical indicator is generated for the plurality of converted weights; and the graphical indicator is shown overlaid to a representation of the target conversion-mesh.

In an example of the method, different weight distributions are calculated with different methods and are displayed to the user for selection; and
wherein the selected method is applied for conversion.

As an example, the different weight distributions derived from the different methods are displayed to the physician for selection.

The visualization might be in form of a color-coded overlay of the target mesh.

For example, the user is a physician or another staff member in an operation room or catheterization laboratory, also known as cathlab.

As an additional example, different weight distributions are only displayed if their differences are above a certain threshold.

In an example of the method, it is also provided the steps of:
- registering the first mesh with the second mesh based on the conversion model and the plurality of the converted weights; and
- registering the second 3D image data and the first 3D image data based on the registering of the first mesh and the second mesh, thereby generating combined image data; and
- presenting the combined image data.
As an example, it is provided the steps of
- registering the first mesh with the second mesh based on the conversion model and the plurality of the converted weights; and
- registering the pre-interventional 3D image data and the interventional image data based on the registering of the first mesh and the second mesh, thereby generating combined image data; and
- presenting the combined image data

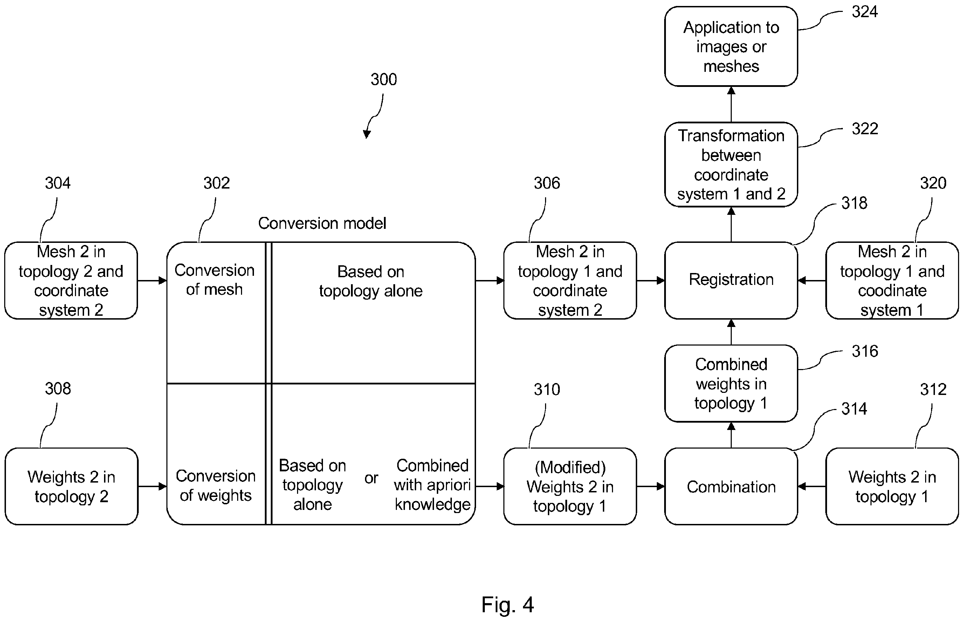

Fig. 4 shows a working scheme 300 of another example for registering second 3D image data to first 3D image data. A conversion model 302 is indicated with a larger frame. In 304, a mesh 2 in a topology 2 and a coordinate system 2 are provided to the conversion model 302 for a conversion of the mesh. Based on topology alone, in 306 this results in a mesh 2 in topology 1 and the coordinate system 2. In 308, weights 2 in topology 2 are provided to the conversion model 302 for a conversion of the weights. Based on topology alone, or combined with apriori knowledge, in 310 this results in, e.g. modified, weights 2 in topology 1. The weights 2 in topology 1 of 310, e.g. modified weights, and weights 2 in topology 1 of 312 are combined in 314 to achieve combined weights in topology 1 in 316. The mesh 2 in topology 1 and the coordinate system 2 of 306 and mesh 2 in topology 1 and coordinate system 1 of 320 together with the combined weights in topology 1 of 316 are registered in 318. A transformation between coordinate system 1 and 2 is provided in 322 and the result of that is provided for application to images or meshes in 324.

Further, a computer program comprising instructions is provided which, when the program is executed by a computer, cause the computer to carry out the method of any of the preceding examples.

As an example, a computer program or program element for controlling an apparatus according to one of the examples above is provided, which program or program element, when being executed by a processing unit, is adapted to perform the method steps of one of the method examples above.

As an example, a computer readable medium having stored the computer program of the previous example is provided.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for registering second 3D image data to first 3D image data, the device comprising:
- a data input (12);
- a data processor (14); and
- an output interface (16);
wherein the data input is configured to:
- provide first 3D image data comprising a first mesh with a first topology;
- provide second 3D image data comprising a second mesh with a second topology; wherein the second topology is different than the first topology; and wherein one of the first mesh and the second mesh is a to-be-converted mesh and the other of the first mesh and the second mesh is a conversion-target mesh;
- provide a conversion model to convert the to-be-converted mesh to the conversion-target mesh; and
- provide a plurality of weights for the to-be-converted mesh;
wherein the data processor is configured to convert the to-be-converted mesh to the conversion-target mesh based on the conversion model; wherein, based on the conversion model, the plurality of weights for the to-be-converted mesh is converted into weights for the conversion-target mesh to thereby generate a plurality of converted weights; and
wherein the output interface is configured to provide the plurality of converted weights for further image data processing.

2. Device according to claim 1, wherein the first 3D image data is different than the second 3D image data; and
wherein a difference is provided for at least one of the group comprising:
- image data resolution;
- time of image acquisition;
- subject's state for image acquisition;
- imaging used for generating the image data; and
- imaging modality used for generating the image data.

3. Device according to claim 1 or 2, wherein the data input is configured to provide the first 3D image data as interventional 3D image data; and to provide the second 3D image data as pre-interventional 3D image data.

4. Device according to one of the preceding claims, wherein the data processor is configured to assess image quality of both the second 3D image data and the first 3D image data separately; and
wherein the data processor is configured to compute an overall weight distribution based on both weight distributions of the second 3D image data and the first 3D image data.

5. Device according to one of the preceding claims, wherein the data processor is configured to derive the plurality of weights from imaging affected parameters of the actual imaging of the second 3D image data and/or the first 3D image data, the parameters comprising at least one of the group of: image quality and field of view.

6. Device according to one of the preceding claims, wherein the data processor is configured to derive the plurality of weights from systematic inter-modality deviations of the imaging modalities.

7. Device according to one of the preceding claims, wherein the data processor is configured to derive the plurality of weights from patient specific circumstances.

8. Device according to one of the preceding claims, wherein the data processor is configured to derive the plurality of weights from different types of the image-related content; and
wherein the data processor is configured to generate a separate set of a plurality of weights for each type for each image category of the second 3D image data and the first 3D image data; and
wherein the data processor is configured to transfer the different sets of the plurality of weights of each image category into a common plurality of weights for each image category.

9. Device according to one of the preceding claims, wherein the data processor is configured to generate a graphical indicator for the plurality of converted weights; and
wherein the output interface is configured to show the graphical indicator overlaid to a representation of the target conversion-mesh.

10. Device according to one of the preceding claims, wherein the data processor is configured to calculate different weight distributions with different methods;
wherein a display () is provided that is configured to display the different weight distributions to the user for selection; and
wherein the data processor is configured to apply the selected method for conversion.

11. Device according to one of the preceding claims, wherein the data processor is configured to:
- register the first mesh with the second mesh based on the conversion model and the plurality of the converted weights; and
- register the second 3D image data and the first 3D image data based on the registering of the first mesh and the second mesh, to thereby generate combined image data; and
wherein the output interface is configured to present the combined image data.

12. A medical imaging system (100) comprising:
- a first imaging modality (102); and
- a device (10) for registering second 3D image data to first 3D image data according to one of the preceding claims;
wherein the first imaging modality provides the first 3D image data.

13. Medical imaging system according to claim 12, wherein it is provided:
- a second imaging modality (104);
wherein the second imaging modality provides second 3D image data; and
wherein the second imaging modality is different than the first imaging modality.

14. A method (200) for registering second 3D image data to first 3D image data, the method comprising the following steps:
- providing (202) first 3D image data comprising a first mesh with a first topology; and providing second 3D image data comprising a second mesh with a second topology; wherein the second topology is different than the first topology; and wherein one of the first mesh and the second mesh is a to-be-converted mesh and the other of the first mesh and the second mesh is a conversion-target mesh;
- providing (204) a conversion model to convert the to-be-converted mesh to the conversion-target mesh;
- providing (206) a plurality of weights for the to-be-converted mesh;
- converting (208) the to-be-converted mesh to the conversion-target mesh based on the conversion model; wherein, based on the conversion model, the plurality of weights for the to-be-converted mesh is converted into weights for the conversion-target mesh thereby generating a plurality of converted weights; and
- providing (210) the plurality of converted weights for further image data processing.

15. Computer program comprising instructions which, when the program is executed by a computer, cause the computer to carry out the method of claim 14.
